# EUROPEAN PATENT APPLICATION

(11) **EP 3 195 898 A1**
(43) Date of publication of application: **26.07.2017**
(21) Application number: 17152454.9
(22) Date of filing: 20.01.2017
(51) Int. Cl.: A61N 1/05, A61N 1/40, A61N 1/32

(54) **A RADIOFREQUENCY AND PLASMA DEVICE FOR VAGINAL LAXITY AND REMODELING**

(30) Priority: 22.01.2016 IT UB20160508
(71) Applicant: Bios S.r.l. Unipersonale, 20090 Vimodrone, Milano (IT)
(72) Inventor: CASALINO, Lorenzo, I-20090 Vimodrone, Milano (IT); CASALINO, Aldo, I-20090 Vimodrone, Milano (IT)
(74) Representative: Rigamonti, Dorotea

(57) **Abstract**

The present invention relates to a device for vaginal remodeling which comprises:
a) a preferably disposable, sterilizable handpiece (101, 201, 301, 401, 601, 701), which is an elliptical or preferably cylindrical applicator, adapted to be inserted in vagina, wherein at least one electrode (102, 202, 302, 402, 602, 702) is positioned either on the outer surface (108, 208, 308, 408, 608) of said handpiece (101, 201, 301, 401, 601) or on the inner surface of said handpiece (701);
b) a machine which emits an RF (Radio Frequency) sinusoidal signal, either continuous or pulsed, such to cause heating in the tissues, or a plasma discharge, having a frequency from 10 kHz to 6 MHz, preferably between 50 kHz and 2 MHz, or between 200 kHz and 6 MHz, and connected to said at least one electrode;
c) temperature sensors, for detecting the temperature, positioned on the handpiece (101, 201, 301, 401) itself;
d) PID algorithm based software for the feedback control of the power delivered on the basis of the detected temperature,
characterized in that the curvature radius of said at least one electrode (102, 202, 302, 402) is equal to that of the handpiece (101, 201, 301, 401) on which it is positioned and is preferably either tubular (102, 202) or annular (302).

## Description

The present invention relates to a device for vaginal remodeling which comprises:
a) a handpiece (101, 201, 301, 401, 601, 701), which is an elliptical or preferably cylindrical applicator, adapted to be inserted in vagina, wherein at least one electrode (102, 202, 302, 402, 602, 702) is positioned either on the outer surface (108, 208, 308, 408, 608) of said handpiece (101, 201, 301, 401, 601) or on the inner surface of said handpiece (701);
b) a machine or apparatus which emits an RF (Radio Frequency) sinusoidal signal, either continuous or pulsed, such to cause heating in the tissues, or a plasma discharge, having a frequency from 10 kHz to 6 MHz, preferably between 50 kHz and 2 MHz, or between 200 kHz and 6 MHz, and connected to said at least one electrode (electric carrier signal);
c) temperature sensors, for detecting the temperature positioned on the handpiece (101, 201, 301, 401) itself;
d) PID algorithm based software for the feedback control of the power delivered on the basis of the detected temperature,
characterized in that the curvature radius of said at least one electrode (102, 202, 302, 402) is equal to that of the handpiece (101, 201, 301, 401) on which it is positioned and is preferably either tubular (102, 202) or annular (302).

### Prior art

The vaginal relaxation syndrome, also known as "wide vagina", is a condition of laxity of the vaginal and pelvic structures, which are subject to tissue relaxation, thus losing the tone and strength, which physiologically distinguish them.

Main cause of the syndrome is vaginal childbirth, above all at a later age: with age, the collagen present in the vaginal structure tissues, responsible for their elasticity, is renewed slower, thus considerably increasing the risk of permanent genital relaxation.

In presence of vaginal structure laxity, either permanent or transient, the first complained problem is related to sexual intercourse. Then, urinary incontinence is also often associated with the vaginal relaxation syndrome. The loss of tone and of voluntary vagina musculature control may cause loss of urine following normal everyday movements (e.g. sneezing or coughing).

The histological examination of a sample of vaginal wall affected by laxity reveals a squamous layered epithelium lining, with a given degree of surface keratinization. A thick connective tissue layer is found under the epithelium. Connective tissue aberrations or deformities have been identified as predominant cause of vaginal laxity.

The connective tissue, typically supporting tissue, is characterized by the presence of cells dispersed in an extracellular matrix, consisting of an amorphous component and a fibrous component. In the vaginal connective tissue, the fibrous component mostly consists of collagen. Vaginal connective tissue defects predispose for pelvic prolapse in the worst cases or vaginal laxity in the best cases. Women with diagnosed POP (Pelvic Organ Prolapse) or cutaneous laxity have shown a lower total collagen content in the supporting pubocervical fascia, together with a weaker collagen matrix.

In Europe, 24 per cent of women suffer from POP.

The following treatments are currently suggested for POP:
- Vaginal or abdominal surgery, which however, in addition to not guaranteeing certainty of results, is generally not desired.
- Use of vaginal devices (pessaries), which are inserted in the vagina and mechanically reduce the prolapse. Vaginal devices are well known for their diversity in terms of shape and size. Some of these devices tend to block the flow of urine completely, and thus the device must be removed from the vagina or must be compressed to remove the pressure applied against the bladder neck when the patient needs to urinate. In an attempt to solve this problem, vaginal devices of special shapes which do not block the bladder neck completely with the device in position have been developed. Examples of these are described in US 6,158,435; US 5,894,842; US 5,771,899, US 4,823,814. However, these devices are generally large and invasive, inconvenient to insert, carry and remove, with low patient satisfaction and *compliance.* US 6,189,535, US 5,611,768 attempt to overcome the drawbacks above with devices the volume of which can be varied as needed by either introducing or releasing the air contained therein.
- Use of devices for promoting connective tissue remodeling, such as those described in WO2007/092610, WO2013/138718. These are devices for treating the vaginal epithelium, which exploit the heat emitted by a source. However, the use of said devices implies some disadvantages, mainly associated with the risk of burns by reaching excessively hot temperatures, making a surface cooling system of the device itself necessary. WO2015/059120 describes an ablative CO₂ laser, which creates micro burns to the vaginal tissue in order to stimulate the production of collagen. A CO₂ laser treatment may or may not be associated with radiofrequency current (RF) by means of filiform electrodes. The solution is however invasive, causing post-surgery problems, because the skin, being damaged, is exposed to infections and complications. US2015297908 (A1) describes a probe from the end of which RF current is delivered, with the drawback of heating small zones at a time, and thus the difficulty of completing the treatment correctly, avoiding superimposed zones and avoiding any zone from being left untreated.

Furthermore, all these treatments are performed manually, are operator-dependent because the operator's presence is required during the entire treatment, given the need to move the applicator on the zone to be treated.

Thus, the need is strongly felt to avail of a device for vaginal remodeling which overcomes the limitations of those described to date.

### Description

The device which is the object of the present invention is a device which, by means of an RF signal, is able to both heat the internal and external vaginal tissues for an adequate time up to reaching and keeping the set temperature, in gradual, non-invasive or ablative manner, and without post-treatment complications, and to generate cold plasma capable of creating micro-holes in the tissues.

The heat or the plasma delivered by said device stimulates the production of new collagen and the recovery of the original tissue tone in effective, painless, pleasant, safe, non-invasive manner, thus not accompanied by side effects, such as bleeding, ablations or abrasions. Advantageously, the device of the present invention does not require the constant presence of an operator.

### Description of the figures

Figure 1: front view of a handpiece according to an embodiment of the present invention.
Figure 2: front view of a handpiece according to a further embodiment of the present invention.
Figure 3: front view of a handpiece according to a further embodiment of the present invention.
Figure 4: front view of a handpiece according to a further embodiment of the present invention.
Figure 5: diagram showing the emission of RF signal from a handpiece in an embodiment according to the present invention.
Figure 6: front view of a handpiece according to two further embodiments of the present invention, panel A and B.
Figure 7: front view of a handpiece according to two further embodiments of the present invention, panel A and B.

### Detailed description of the invention

The device according to the present invention comprises a handpiece (101, 201, 301, 401, 601, 701), which is an applicator (101, 201, 301, 401, 601, 701), the shape of which is modeled to follow the body cavity in which it is inserted, i.e. the vagina, at least one electrode for emitting RF or plasma energy, connected to a machine for generating RF or plasma energy, analog or digital optical sensors for detecting the temperature and PID algorithm based software for the feedback control of the delivered power on the basis of the detected temperature.

Said applicator is elliptical or, preferably, cylindrical. Said applicator (101, 201, 301, 401, 601, 701) has an outer contact surface (108, 208, 308, 408, 608) with the epithelium and, in an alternative embodiment, is hollow and has an inner surface. At least one electrode (102, 202, 302, 402, 602) is positioned on the outer surface (108, 208, 308, 408, 608) of said applicator (101, 201, 301, 401, 601), said at least one electrode being connected to a machine which emits RF current.

Said analog or digital optical sensors for detecting the temperature are positioned inside the handpiece.

Said device is characterized in that the curvature radius of said at least one electrode (102, 202, 302, 402) is equal to that of the handpiece (101, 201, 301, 401) on which it is positioned. In some embodiments, said at least one electrode (102, 202, 302) is tubular or annular and is positioned on the circumference of said handpiece. Alternatively, said electrode (402) is positioned longitudinally on the surface of said handpiece (401). Said handpiece is preferably made of rigid material or biocompatible silicone rubber, and has a diameter preferably comprised between approximately 10 and approximately 40 mm and a length which varies preferably between 70 and approximately 140 mm. Said at least one electrode positioned on said handle is a resistive or capacitive electrode (102, 202, 302, 402, 602, 702). Where said electrode is an insulated electrode, thus with capacitive effect, the insulation is given by biocompatible material with high dielectric power.

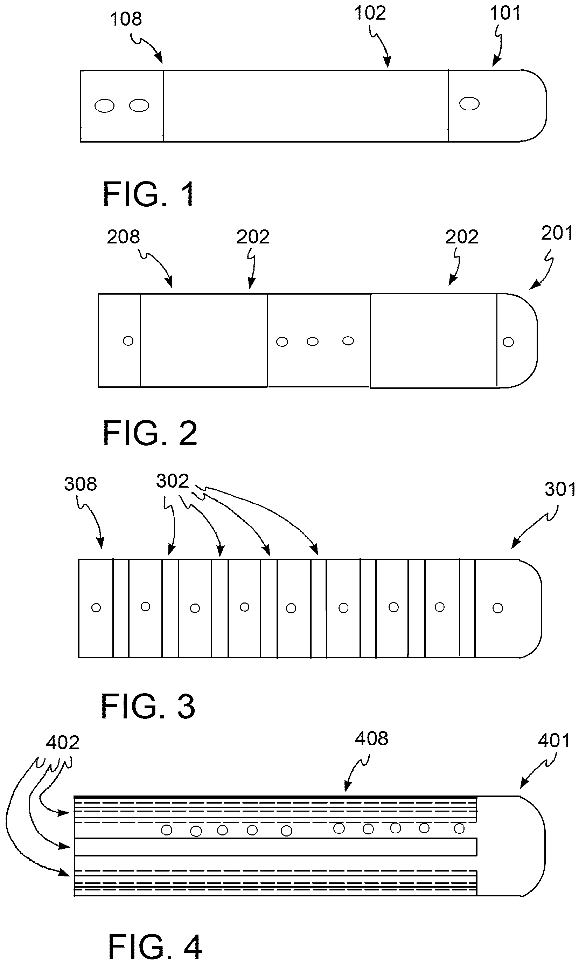

In an embodiment, diagrammatically shown in Figure 1, said handpiece 101 comprises a single electrode (102) and is a monopolar emission handpiece. In said device, the current generated by an RF current generating machine external to the handpiece itself passes through said electrode and the circuit is closed by a non-insulated return electrode (counter electrode), which is a conductive rubber or metal plate to be positioned on the body of the individual concerned by the treatment, preferably under the gluteus, or a cylinder to be held in hand. The capacitive mode allows to heat the tissues characterized by low resistance to current, such as connective tissue, in specific manner. In this embodiment, said capacitive electrode (102) is tubular and covers nearly the entire outer surface 108 of said handpiece (101).

In a further embodiment, diagrammatically shown in Figure 2, said handpiece (201) comprises two tubular electrodes (202) on its outer surface (208). In this embodiment, the handpiece is a bipolar emission handpiece and does not need a return plate.

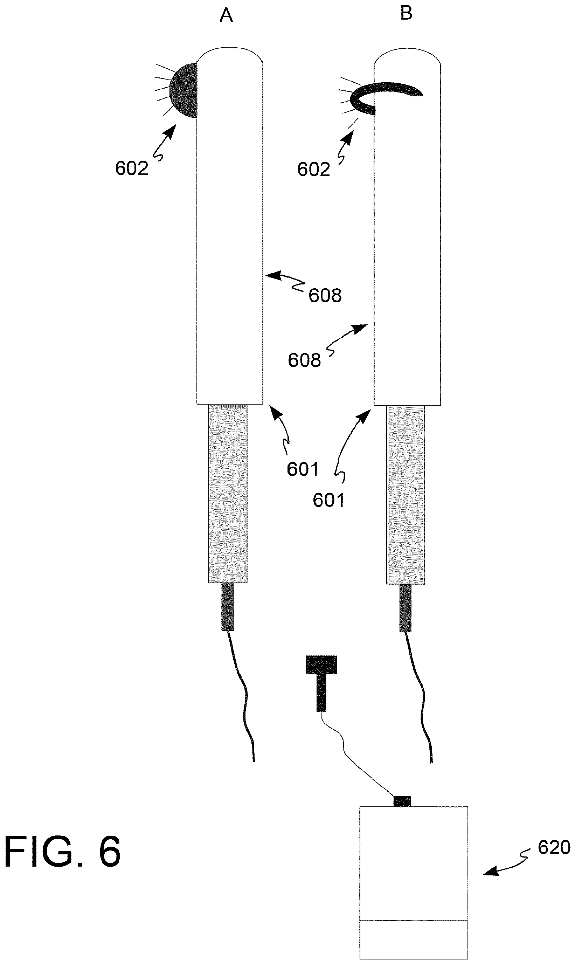

In alternative embodiments, Figure 3 and Figure 4, said electrodes are more than two and said handpieces are multipolar emission handpieces. Said two or more electrodes (302) are annular, so as to surround said handpiece, or are strips (402) arranged in parallel along said handpiece, with a radius of curvature equal to the radius of curvature of said handpiece. Said electrodes are, in turn, separate and thus mutually insulated, and each one is electrically insulated (capacitive effect). This effect is obtained either with anodizing treatments of adequate thickness, or with hard oxide treatment, or by coating with biocompatible material, such as, for example, ceramic, Rilsan or Nylon. In a further embodiment, said one or more electrodes on said handpiece are resistive electrodes, and thus not insulated. In a further embodiment, diagrammatically shown in figure 6, the electrodes (602), either circular (panel A) or half-ring-shaped (panel B), are arranged outside the handpiece. These are monopolar and the handpiece is free from return (or ground) electrode. In this embodiment, the electrodes generate a cold plasma discharge.

The handpiece of the device according to the present invention is connected to a machine capable of generating RF current from 10 kHz to 6 MHz, preferably between 50 kHz and 2 MHz or between 200 kHz and 6 MHz. The connection is made with a composite electric cable, cable which also contains optical fibers for detecting the temperature and, optionally, cables for the electric signals. Preferably, said cable is inserted on said handpiece by means of an electric connector. Said electric connector can be protected so as to allow, once said cable has been disconnected, the sterilization of the handpiece in autoclave, or said handpiece can be eliminated because it is disposable. Said electrodes (102, 202, 302, 402, 602) emit RF current which heats the vaginal tissue with which they are put into contact. Said handpiece also comprises temperature sensors, such as for example optical sensors or optical fibers, which report the IR energy detected on the contact surface to the machine. Said value is reported to the machine, which converts it by means of pyrometers into a temperature value. Where the handpiece is a monopolar handpiece, in the embodiment described in Figure 1, the temperature control is obtained with the PID algorithm, thus modulating the waveform or delivering RF energy at constant power but alternating emission cycles and emission interruption cycles, i.e. by varying the *duty cycle.* In the bipolar or monopolar embodiments, the temperature control is obtained by means of the PID algorithm, by means of detections made in situ by the sensors or optical fibers present on the handpiece which report the infrared energy (IR) detected on the contact surface to the emitting machine and, additionally, with a particular scanning emission of the energy obtained by virtue of the presence of multiple electrodes, as described in greater detail in the following paragraphs.

The device of the present invention allows a fine adjustment of the energy emissions, by virtue of the control performed by means of the PID algorithm with which the device is provided, and the possibility of modulating the energy emission power by means of a modulating electric signal and/or of alternating emission cycles and off cycles.

The device can autonomously adjust the emissions so that the temperature identified as optimal is kept in the action site and the contact surface with the epithelium never exceeds the temperature of 45°C. This means that the applicator does not need to be moved manually to avoid the maximum permitted temperature from being exceeded but can be left in situ for the entire duration of the treatment.

In an alternative embodiment, said handpiece is hollow and perforated, preferably has a series of holes arranged on its entire surface. Said hollow handpiece has an opening at the proximal end, the one opposite to the one which is inserted in vagina. Said open proximal end allows the introduction of a possible composition of pharmaceuticals or cosmetic products into said handpiece. Said hollow handpiece may be provided with a plunger which is inserted in said open proximal end and on which it is possible to act so as to adjust the release in situ of said composition contained in the handpiece itself.

A vaginal remodeling method is also described which comprises:
a) making available a handpiece according to the present invention;
b) introducing said handpiece in vagina;
c) activating the emission of RF current;
d) continuing the treatment until the entire area to be treated is uniformly exposed to the treatment itself.

Using a monopolar handpiece, according to the embodiment shown in Figure 1, the entire inner surface of the vagina may be treated uniformly, with a penetration of the action variable according to the length of the electrode. In this embodiment, the return electrode is external, may be positioned, for example, behind the individual's back. Once the working temperature defined as optimal has been reached, the RF current continues to be emitted in continuous manner, modulated by means of a modulating electric signal or in cycles, i.e. by varying the *duty cycle.* It is the emission power that varies so as to keep the temperature defined as optimal constant.

By using a bipolar handpiece, according to the embodiment shown in Figure 2, the two insulated electrodes on the handpiece work as emission electrode and as return electrode. This offers the advantage of being able to operate exclusively in the zone comprised between the two electrodes, and the depth of action is correlated with the size and the distance between the electrodes. Also in this embodiment, once the desired temperature has been reached, the emission may continue to be delivered either continuously, modulated or in cycles, so as to keep the temperature constant.

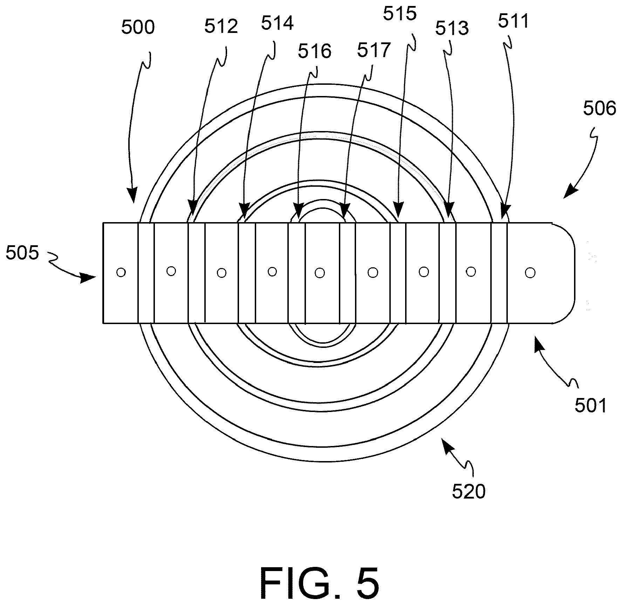

In a further embodiment, where a multipolar handpiece is used, the capacitive or resistive multipolar emission occurs simultaneously from multiple pairs of electrodes and this results in the possibility to treat a thickness at different depths at the same time, as shown in figure 5. By way of example, a handpiece 501 comprises 8 annular electrodes, 510, 511, 512, 513, 514, 515, 516, 517. In an embodiment, the electrode 510, positioned at the proximal end 505 of said handpiece, is placed in circuit with the electrode 511, positioned at the distal end 506. The second proximal electrode, 512, forms a circuit with the second distal electrode 513, the third proximal electrode, 514, forms a circuit with the third distal electrode 515, the fourth proximal electrode, 516, forms a circuit with the fourth distal electrode 517. The mutually more distant electrodes, the proximal electrode 510 and the distal electrode 511 in the case in the example, act with a range of action which allows a deeper action than that which is obtained by forming a circuit with electrodes gradually closer to one another, as exemplified in the action zones 520 diagrammatically shown in Figure 5 itself. In this same embodiment, the pairs of electrodes are activated either simultaneously or, alternatively, singularly for a short time, so as to reach the optimal temperature in gradual manner.

With the same multipolar handpiece, such as the handpiece with 8 electrodes illustrated by example above, a further method is that which consist in a fractioned emission, in which the working frequency is fixed, i.e. the electric carrier signal is at fixed frequency, at 4 Mhz by way of non-exhaustive example, and it is the *duty cycle* that varies, i.e. the percentage of time during which the signal is active, e.g. between 20 and 80%. Alternatively, it is the electric signal itself to be modulated, by means of a modulating electric signal, where the frequency of the modulator is variable from 0.1 to 200 Hz, preferably between 0.1 and 100 Hz, even more preferably between 1 and 100 Hz. Fine control of the temperature generated in the action site is possible by virtue of the many possibilities offered by the emission adjustment system. The different combination of adjustment systems, some exemplified by way of non-exhaustive examples, consist in combining fixed frequency of the carrier electric signal with on/off cycles, or carrier electric signal modulated by a modulating electric signal, makes available very effective working protocols specific for the treatment to be implemented, e.g. tissue flaccidity, or the formation of new collagen on dermal level.

In a further embodiment, said method is associated with a conductive composition of the RF current, which comprises hydrating active principles. Preferably, said composition comprises hyaluronic acid and is in form of cream or gel. Said composition is applied on the surface of said handpiece before the latter is inserted. Alternatively, using the embodiment which provides a hollow and perforated handpiece, the handpiece is filled with said composition, preferably in form of gel, and, with the handpiece inserted, the composition is released through the holes by operating the plunger.

In a further embodiment, said method is advantageously implemented by using handpieces provided with electrodes of particular shape and arrangement, so that said electrodes generate a cold plasma discharge following the activation of the specifically made power board. The power generator is able to create a high voltage and current density, such to ionize the surrounding air, on the surface of the electrodes. As there is no return (or ground) plate, and thus there is no current which closes the circuit through the body, cold plasma micro-discharges are created with a temperature either lower than or equal to 50°C near said electrodes either outside or inside the handpiece.

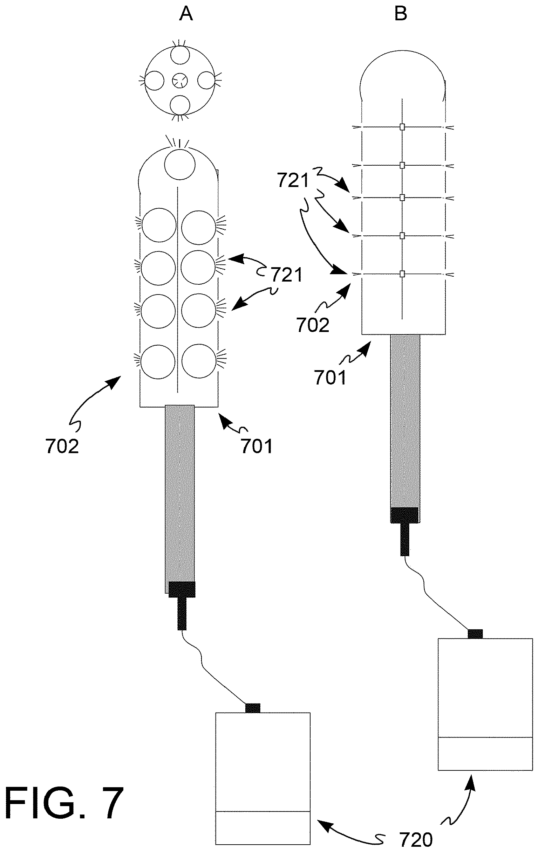

By way of example, the electrodes 602 are positioned outside the handpiece and are semi-spherical or ring-shaped, Figure 6, panel A and B. Alternatively, the electrodes 702 are positioned inside the handpiece, Figure 7.

Where the electrodes are "pointed", as diagrammatically shown in Figure 7, panel B, the generated micro-plasma acts on a punctiform area, which is not extended as in the case of the ball or the ring, creating cold and painless punctiform micro-burns, i.e. necrotic micro-zones surrounded by healthy tissue which is stimulated to produce elastin and collagen in order to restore the necrotized area rapidly. One or more electrodes distributed in the handpiece may be activated either simultaneously or individually, and in this case the operation may be carried out in random or sequential manner. In another form, the electrode is either a circular section ring which surrounds the head of the handpiece or only one half, or a sphere (Figure 6). By rubbing the ring or the sphere on the mucosa, the plasma creates micro-holes with greater extension and lesser depth than that observed with pointed electrodes.

Where the emission of the positioned electrodes is monopolar and free from return plate, a series of advantages are observed: firstly, as no electric current circulates in the patient's body, the treatment is painless, because the nerve terminals are not involved. Furthermore, it is safe for patients who may have heart complaints, and electric risks are also excluded. Without the return electrode, the RF energy is concentrated at the end of the electrodes, thus ionizing the air causing micro-holes of variable depth, diameter and number on the skin surface, as with fractional laser, but at a temperature of about 45°C. This allows to perform absolutely non-invasive treatments without anesthesia and without flushing.

In the embodiment which provides a return electrode to be positioned on the patient's body, there is passage of current at very low amperage in the patient's body. This is for the purpose of being able to measure the tissue impedance in the zone to be treated before the treatment so as to be able to set the operative parameters correctly. The operator, by means of the handpiece of the present invention with electrodes positioned inside, may decide to maintain the handpiece in a stationary position in vagina, and by means of the commands of the control electronics may decide to emit plasma from multiple electrodes either simultaneously or individually in random manner.

In the embodiment of the handpiece with electrodes positioned outside the surface, the operator manually operates on the tissues both inside and outside vagina, by rubbing the zone to be treated with the electrodes.

To indicate the position of the electrodes to the operator and to provide indications on their position during the step of extracting and rotating, the handpieces can be provided with appropriate indicators printed on the handle or with position sensors.

The handpiece may contain the high-voltage RF signal generator power board, and thus can be powered by battery or by mains power via a medical power supply unit, but in the preferred embodiment the handpiece will be disposable, while the power board together with the control body could be inserted in an apparatus (620, 720) typically fixed to the wrist. In this case, the apparatus will be powered by rechargeable lithium polymer batteries or from the mains by means of a medical power supply unit, while the disposable handpiece will be connected to the apparatus fixed to the wrist and managed thereby.

The apparatus may be managed by the user by means of a touchscreen display, and also by means of a tablet in communication with the apparatus fixed to the wrist in wireless manner via a Bluetooth receiver board.

The control board and the tablet will be provided with software specifically designed to facilitate correct operation and parameter selection. Thus, the battery state, the power level, the impedance, the temperature, the timer, the operative mode, the alarms, etc. may be advantageously shown on said display of said apparatus.

The surface of the handpiece is electrically insulated and biocompatible.

Analog or digital temperature sensors or optical sensors or pyrometers may be arranged in the handpiece for controlling the tissue temperature.

The parameters which are advantageously applied to the handpiece using the plasma electrodes according to the present invention are the following:
Continuous or pulsed preferably sinusoidal waveform settable by the operator. In pulse mode, the pulse time will be adjustable from 50 to 500 µs, while the pulse frequency will be adjustable from 1-200 Hz. In continuous mode, the *duty cycle* can be adjusted.

Low amperage current, preferably 0.5 A.
RF sinusoidal wave frequency: 50-2.000 kHz

Transformer voltage up to 3kV.

Constant power generator, settable from 0.3 to 3W.

The emission of the plasma delivered by means of electrodes positioned in the handpiece occurs without return electrode applied to the patient.

The emission occur either simultaneously from all the electrodes positioned in the handpiece or individually from each electrode in sequence or random manner.

Optionally, the handpiece is provided with light, preferably with LEDs, to illuminate the working area.

Preferably, the apparatus is connected to a tablet with a touchscreen display with chosen functions, e.g. selected from: use protocols with preset parameters associated with images of degenerative skin conditions which could be sent by the control electronics via Bluetooth, showing the charge state of the battery, with acoustic signal to indicate, for example, the last 5 minutes of operation, e.g. prolonged beep every minute until expiry, showing remaining time of use at the desired power, showing the minutes of operation from start of treatment, with reset, showing detected impedance, showing symbols envisaged by medical standards, continuous or intermittent acoustic signal emission according to the emission mode, showing steady or blinking LED according to the emission mode, warning signals, e.g. interruption of the connection with electrodes, of interruption of the operation, e.g. when the batteries are connected to the power supply unit for recharging, and other. The treatment performed with any one of the handpieces according to the present invention is very safe and non-invasive.

The temperature detection, carried out by means of optical sensors, offers the advantage of being more reliable and accurate than the detections made with electronic sensors. Furthermore, they do not require electricity, thus making the device safer.

In the device according to the present invention, the temperature is controlled surprisingly by combining the PID control system with electrodes positioned on the handpiece and optionally a scanning emission system. The combination of the elements allows accurate and punctual control independently from the operator. Consequently, the temperature of the contact surface is kept constant for even very long time without needing to move the handpiece to avoid overheating. The treatment time is about 10-15 minutes in average. With the device of the present invention, it is possible to insert the handpiece and maintain it in the same position for the entire duration of the treatment, where the temperature of the contact surface is kept optimal with the adjustment systems indicated above.

A further advantage of the device and method described herein is related to the use of RF, the effect of which, unlike laser, for example, does not depend on skin color or conditions, such as dehydration, cracking or other.

Many are the applications of plasma in medicine today. This energy form may be used also for non-ablative treatments, such as tissue regeneration, tissue and vaginal rejuvenation, tissue sterilization, tissue bio-stimulation, micro-capillary coagulation etc. The use of low power cold plasma creates micro-holes in the skin at a temperature lower than 50°C. This allows to perform absolutely non-invasive treatments without anesthesia, even in the summer, without flushing. The percentage of surface concerned by the micro-holes is minimal, and this allows for most of the skin to be intact, thus constituting a reserve for quickly repairing the micro-holes, with distension of the tissue and increase of tone. Varying the diameter of the micro-hole, e.g. by varying the size of the tip of the pointed electrode, may concern a greater percentage of the treated surface, passing from the fractional effect to *resurfacing,* which allows to remove a surface layer of the epidermis. In this manner, it is possible to very effectively treat acne scars, micro-wrinkles and other blemishes characterized by surface irregularities typical of the vaginal tissue. This is a valid alternative to *peeling* and dermoabrasion. Furthermore, unlike *needling* and other methods which overcome the skin barrier, since cold plasma is a disinfection method in itself, the micro-holes are sterile, thus without the danger of conveying the bacteria normally present on the skin surface and above all in the vaginal environment into depth. Furthermore, considering the action temperature above 45°C, instead of 170-200°C, there will be no flushing nor any type of discomfort. Indeed, cold plasma has been approved for some time by the FDA for disinfection because it can penetrate also beyond bacterial walls, viral capsules and any other type of pathogen microorganisms (fungi, etc.). Another effect is that of stimulating the fibroblasts to produce collagen and elastin to repair the micro-holes. By using substances such as boswellic acid in cream (which penetrates into the micro-holes), the formation of elastic fibers is stimulated, so that tissue reacquires elasticity and the features of a younger tissue. So, with cold plasma it is possible to obtain the bio-regeneration and the bio-stimulation of the vaginal tissue. The result will be rejuvenation, reduction of skin slackening and equally important results in flattening wrinkles and in skin tone.

## Claims

1. A vaginal remodeling device which comprises:
a) a preferably disposable, sterilizable handpiece (101, 201, 301, 401, 601, 701), which is an elliptical or preferably cylindrical applicator, adapted to be inserted in vagina, wherein at least one electrode (102, 202, 302, 402, 602, 702) is positioned either on the outer surface (108, 208, 308, 408, 608) of said handpiece (101, 201, 301, 401, 601) or on the inner surface of said handpiece (701);
b) a machine which emits an RF (Radio Frequency) sinusoidal signal, either continuous or pulsed, or a plasma discharge, having a frequency from 10 kHz to 6 MHz, preferably between 50 kHz and 2 MHz or between 200 kHz and 6 MHz, and connected to said at least one electrode (electric carrier signal);
c) temperature sensors, for detecting the temperature positioned on the handpiece (101, 201, 301, 401) itself;
d) PID algorithm based software for the feedback control of the power delivered on the basis of the detected temperature,
**characterized in that** the curvature radius of said at least one electrode (102, 202, 302, 402) is equal to that of the handpiece (101, 201, 301, 401) on which it is positioned and is preferably either tubular (102, 202) or annular (302).

2. A device according to claim 1, wherein said at least one electrode (102, 202, 302) is either tubular or annular and is positioned on the circumference of said handpiece.

3. A device according to claim 1, wherein said at least one electrode (402) is positioned longitudinally on the surface of said handpiece (401).

4. A device according to any one of the claims from 1 to 3, wherein said at least one electrode is a capacitive electrode, insulated with biocompatible material having high dielectric power.

5. A device according to any one of the claims from 1 to 3, wherein said at least one electrode is a resistive electrode.

6. A device according to any one of the claims from 1 to 5, wherein said at least one electrode is either a sphere-shaped or half-ring-shaped electrode (602) positioned externally on the surface of the handpiece or is a pointed or a sphere-shaped electrode positioned inside the handpiece.

7. A device according to any one of the claims from 1 to 6, wherein said temperature sensors are optical sensors or digital or analog optical fiber sensors.

8. A device according to any one of the claims from 1 to 7, wherein said machine which emits RF current modulates the emission energy waveform by means of a modulating electrical signal and/or delivers RF energy at constant power by alternating emission cycles and emission interruption cycles, thus varying the *duty cycle.*

9. A device according to any one of the claims from 1 to 8, wherein the frequency of said modulating electric signal is comprised between 0.1 and 200 Hz, preferably between 0.1 and 100 Hz, even more preferably between 1 and 100 Hz and said *duty cycle* varies between 20 and 80%.

10. A device according to any one of the claims from 1 to 9, wherein said machine is preferably positioned on the wrist of the operator's hand and said RF current is low amperage current, preferably 0.5 A and said frequency is comprised between 50 and 2000 kHz, the voltage of the transformer is up to 3 kV, and the generator delivers a constant power, from 0.3 to 3 W and said electrodes emit cold plasma.

11. A device according to any one of the claims from 1 to 10 which does not comprise any return electrode or ground.

12. A device according to any one of the claims from 1 to 11, wherein said outer surface in contact with the epithelium reaches a maximum temperature of 45°C.

13. A device according to any one of the claims from 1 to 12, wherein said applicator is hollow and has one or more holes on the surface and an opening at the proximal end, opposite to the end which is inserted in vagina.

14. A device according to claim 13, wherein a composition which comprises pharmaceuticals and/or cosmetics is contained in said cavity and, optionally, a plunger inserted in said opening at the proximal end.
